(19) Europäisches Patentamt
European Patent Office
Office européen des brevets

(11) Numéro de publication: 0 349 373
A2

(12) # DEMANDE DE BREVET EUROPEEN

(21) Numéro de dépôt: 89401699.7

(22) Date de dépôt: 16.06.89

(51) Int. Cl.5: C 07 B 37/04
C 07 B 43/02, C 07 B 45/04,
C 07 C 49/76, C 07 F 7/08,
C 07 C 45/00

(30) Priorité: 27.06.88 FR 8808582

(43) Date de publication de la demande:
03.01.90 Bulletin 90/01

(84) Etats contractants désignés:
AT BE CH DE ES FR GB GR IT LI LU NL SE

(71) Demandeur: RHONE-POULENC CHIMIE
25, quai Paul Doumer
F-92408 Courbevoie Cédex (FR)

(72) Inventeur: Bennetau, Bernard
3, rue Paul Verlaine
F-33740 Ares (FR)

Dunogues, Jacques
22, avenue du Président Poincaré
F-33400 Talence (FR)

Krempp, Michèle
8, rue de Kertzfeld
F-67230 Benfeld (FR)

(74) Mandataire: Vignally, Noel et al
Rhône-Poulenc Chimie Service Brevets Chimie 25 Quai Paul Doumer
F-92408 Courbevoie Cédex (FR)

(54) **Procédé de préparation de dérivés ortho-substitués du fluoro- ou des alkylbenzènes.**

(57) La présente invention concerne un procédé de préparation de dérivés ortho-substitués du fluoro- ou des alkylbenzènes par passage par un intermédiaire disilicié, fonctionnalisation du dérivé et désilylation.

Elle permet d'atteindre des dérivés ortho-substitués à partir de composés de départ essentiellement para-directeurs.

EP 0 349 373 A2

**Description**

## PROCEDE DE PREPARATION DE DERIVES ORTHOSUBSTITUES DU FLUORO- OU DES ALKYLBENZENES

Le présente invention concerne un nouveau procédé de substitution en ortho de dérivés benzéniques monosubstitués par un groupe ortho- et para-directeur. Elle concerne plus particulièrement la préparation de fluoro et d'alkylbenzènes monosubstitués en ortho par un groupe acyle, halogéno, sulfonyle.

Il est connu depuis longtemps que les dérivés du type fluorobenzène ou alkylbenzènes, lorsqu'ils sont soumis à une réaction de substitution électrophile, donnent presque exclusivement des dérivés para-substitués. Or, certaines synthèses chimiques exigent la présence de dérivés ortho-substitués par exemple le fluorobenzène ortho-substitué. Les voies classiques de la chimie ne permettent pas d'arriver à de tels dérivés de manière directe. La chimie est donc toujours à la recherche d'une méthode simple de synthèse de dérivés mono-substitués en ortho du fluorobenzène ou des alkylbenzènes, à partir de ces substrats eux-mêmes.

Il a été découvert de façon tout à fait surprenante que le fluorobenzène pouvait être fonctionnalisé en position ortho, par voie organosilicique, selon le processus réactionnel suivant :

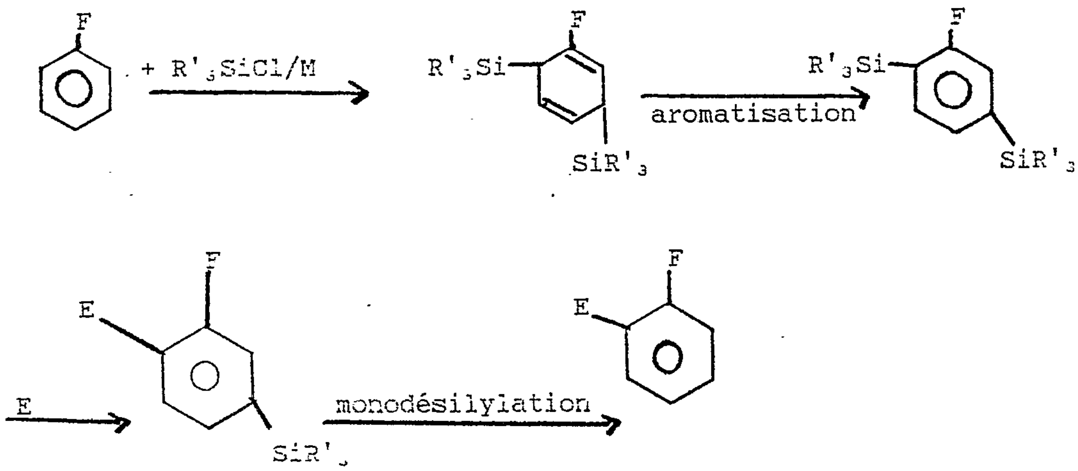


Le procédé de la présente invention consiste à partir de fluoro- ou d'alkylbenzènes à mettre en contact dans une première étape cette matière première avec un halogénure de trialkylsilane en présence de magnésium ou d'un métal alcalin (M) ; dans une deuxième étape à aromatiser le dérivé obtenu à la première étape dans une troisième étape, on substitue en ortho le dérivé obtenu à la deuxième étape ; dans une quatrième étape, on effectue la désilylation du composé obtenu à la troisième étape.

La première étape du procédé est réalisée de manière connue par condensation sur le fluoro- ou un alkylbenzène d'un halogénure de trialkylsilyle, de préférence un chlorotrialkylsilane, dans un solvant aprotique polaire tel que notamment le tétrahydrofuranne, en présence de magnésium ou d'un métal alcalin, par exemple le lithium ou le sodium à basse température. On obtient l'alkyl- ou le fluoro bis (trialkylsilyl-)-3,6 cyclohexadiène -1,4 selon la réaction :

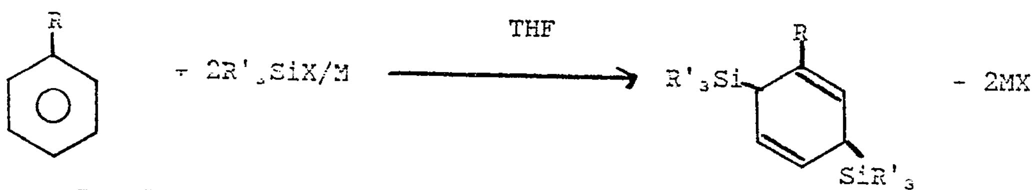


avec R = alkyle ou fluor,
R′ = méthyle ou éthyle,
X = chlore,
M = Mg, Na ou Li

On utilise de préférence au cours de cette étape le lithium finement granulé dans du tétrahydrofuranne anhydre maintenu à une température de préférence comprise entre 0 et 5°C.

La deuxième étape du procédé qui consiste à aromatiser le cyclohexadiène obtenu à la première étape est réalisée au moyen d'un agent oxydant qui peut être le soufre ou l'air, ceci n'étant pas limitatif.

La troisième étape du procédé consiste à fonctionnaliser le produit disilylé obtenu à la deuxième étape. Cette fonctionnalisation se fait par échange entre un agent électrophile et le groupe $-SiR'_3$ situé en ortho. Cet échange peut se faire avec l'ensemble des agents électrophiles tels que par exemple :

- les halogénures d'acides carboxyliques ou sulfoniques
- les anhydrides d'acides carboxyliques ou sulfoniques ou d'autres agents tels que

- le brome,
- ICl,
- l'hypochlorite de calcium,
- le chlorosulfonate de triméthylsilyle,
- les tétrafluoroborates de nitronium,
- le chlorure de tertiobutyle.

La fonctionnalisation par les agents d'acylation est effectuée de préférence en présence d'un acide de Lewis.

L'acide de Lewis est choisi notamment parmi le chlorure d'aluminium, le trifluorure de bore, le tétrachlorure de titane et le tétrachlorure d'étain.

La fonctionnalisation du dérivé disilicié est réalisée dans de nombreux solvants organiques à l'exclusion des solvants basiques. On peut citer à titre d'exemples : les alcanes, le disulfure de carbone, les nitroalcanes, les nitriles, les solvants haloaromatiques nitroaromatiques, ainsi que tous les solvants chloroaliphatiques.

La quatrième étape du procédé objet de la protection demandée consiste à réaliser la désilylation du dérivé obtenu à la troisième étape. La stratégie s'applique à la fois aux dérivés du fluoro- ou des alkylbenzènes.

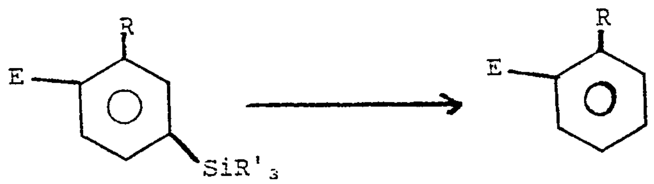

1. Selon un premier procédé cette étape de désilylation est réalisée de préférence en présence d'une base. Cette base est choisie notamment parmi les fluorures alcalins ou alcalino-terreux, les alcoolates, les hydroxydes alcalins ou d'ammonium, les ammonium quaternaires.

On peut citer parmi les fluorures alcalins :
- le fluorure de sodium,
- le fluorure de potassium,
- le fluorure de césium.

On peut aussi citer :
- le difluorure de calcium,
- les fluorures d'ammonium quaternaire,
- les bifluorures alcalins,
- les fluorures de phosphonium ...

On peut citer parmi les alcoolates :
- l'éthylate de sodium,
- le tertiobutylate de potassium.

2. Selon un deuxième procédé, on préfère utiliser comme agent de désilylation un acide fort tel que l'acide trifluoroacétique ou l'acide trifluorométhane sulfonique.

Le solvant de la quatrième étape de désilylation est choisi notamment lors de l'utilisation de bases comme agent de désilylation (point 1, ci-dessus) parmi les solvants aprotiques polaires. On peut citer parmi ceux-ci :
- le diméthylacétamide (DMA),
- le diméthylformamide (DMF),
- la N- méthylpyrrolidone-2 (NMP),
- le diméthylsulfoxyde (DMSO).

Du point de vue conditions réactionnelles, il est particulièrement avantageux de réaliser cette réaction à une température comprise entre la température ambiante et le reflux du solvant.

Lors d'une désilylation dans les conditions indiquées au point 2 à l'aide d'acide trifluoroacétique, le solvant est choisi de préférence parmi les solvants aromatiques et aliphatiques éventuellement halogénés.

Le produit obtenu est extrait du milieu réactionnel par toute technique connue de l'homme de l'art et notamment par extraction à l'aide de solvants.

Parmi les produits obtenus dans le cadre de la présente invention, on peut citer notamment :
- l'iodo-2 éthylbenzène,
- l'éthyl-2 benzènesulfonate de sodium,
- l'acétyl-2 éthylbenzène,
- l'acétyl-2 fluorobenzène,
- le sénécioyl-2 fluorobenzène,
- le benzoyl-2 fluorobenzène,
- l'iodo-2 fluorobenzène,
- le bromo-2 fluorobenzène.

La présente invention va être plus précisément décrite à l'aide des exemples suivants qui ne doivent en aucun cas être considérés comme limitatifs de l'invention.

EXEMPLE 1 - FONCTIONNALISATION EN ORTHO DU FLUOROBENZENE

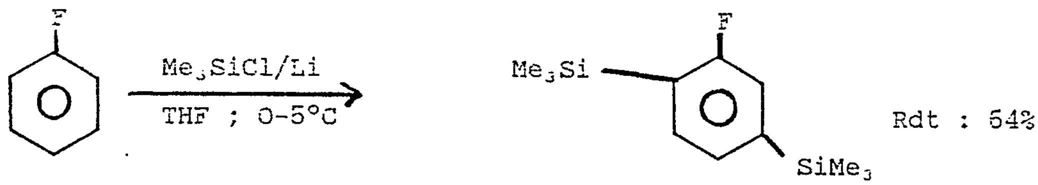


Appareillage :

Il est constitué d'un ballon de Grignard de 500 ml à 3 tubulures, muni d'une agitation magnétique, d'une gaine thermométrique, d'une ampoule à brome isobare et d'un réfrigérant ascendant relié à une colonne à chlorure de calcium. Le réacteur est équipé de façon à travailler sous balayage d'argon.

Mode opératoire :

A une suspension de 3,81 g (0,55 mol) de lithium, finement granulé, dans 225 ml de THF, déperoxydé anhydre, et 60 g de $Me_3SiCl$, fraîchement distillé, on ajouté, goutte à goutte, avec agitation, 14,4 g (0,15 mol) de fluorobenzène en refroidissant de telle manière que le milieu réactionnel soit maintenu entre 0 et 5°C. L'addition terminée, l'agitation est poursuivie 24 heures en maintenant la température vers 5-10°C. La réaction terminée, le Li restant et LiCl sont filtrés et le filtrat évaporé sous le vide de la trompe à eau à 30°C. Dans le cas (le plus fréquent) où LiCl précipite à nouveau, on ajoute 150 ml de pentane sec qui achève la précipitation de LiCl. Après filtration et évaporation du filtrat, on recueille par distillation le produit attendu ($Eb_{0,5\ mm}$ = 76-78°C; F = 42-44°C), avec un rendement de 64 %.

1.2. - AROMATISATION DU DERIVE CYCLOHEXADIENIQUE

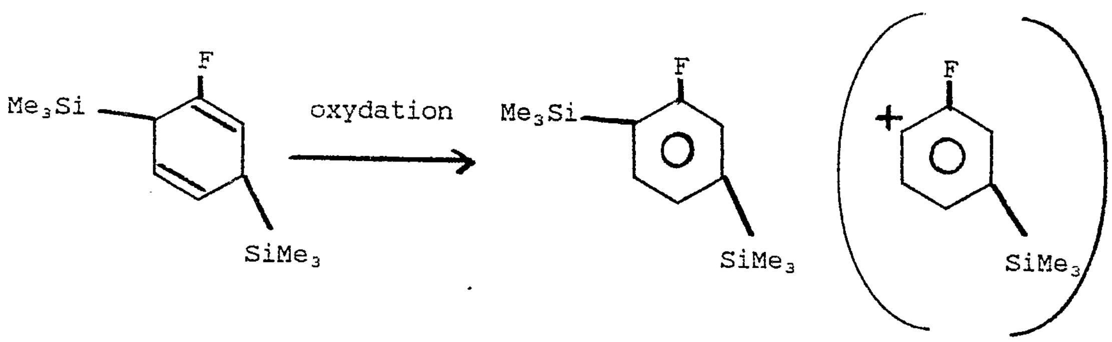


1.2.1 - Aromatisation au soufre

Appareillage :

Ballon de 100 ml muni d'un réfrigérant à reflux et d'une agitation magnétique.

Mode opératoire :

On mélange 15,0 g (62 mmole) de diène avec 7,0 g (220 mmole) de soufre dans 45 ml de toluène. On chauffe au reflux du toluène pendant 18 heures. On évapore le toluène, puis en reprend avec 100 ml de pentane et on filtre afin d'éliminer le soufre en excès. Après évaporation du solvant, le résidu est recristallisé dans l'éthanol. On obtient 7,9 g de cristaux (rendement 53 %). $T_F$ = 91°C (EtOH).

1.2.2 - Aromatisation à l'air

Appareillage :

Erlenmeyer de 1 l irradié par une lampe visible de 150 W, refroidi par un ventilateur.

Mode opératoire :

Dans un Erlenmeyer de 1 l, on introduit 20,0 g (82 mmole) de diène ; après l'avoir bouché, on l'irradie pendant 11 jours en éliminant régulièrement l'eau oxygénée formée. On recristallise le bis(triméthylsilyl)-2,5

fluorobenzène dans l'éthanol ; 14,2 g (72%).

## 1.3 - FONCTIONNALISATION DU BIS(TRIMETHYLSILYL)-2,5 FLUOROBENZENE

### 1.3.1 - Réactions d'acylation

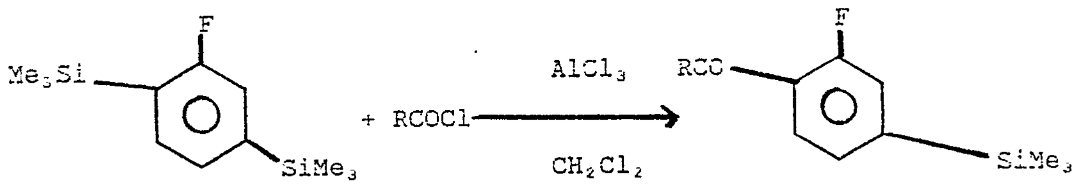


Appareillage :

Ballon de 100 ml, à tubulaire latérale, sous balayage d'argon, muni d'une ampoule isobare et d'une agitation magnétique.

Mode opératoire :

A 1,22 g (9,2 mmol) de chlorure d'aluminium (préalablement dégazé sous vide) en solution dans 10 ml de chlorure de méthylène sec, sont ajoutés, à 0°C, 9,2 mmol de chlorure d'acide fraîchement distillé. L'agitation est ensuite poursuivie à 0°C pendant 15 mn.

Le complexe étant à la température convenable, 2 g (8,3 mmol) de dérivés disiliciés en solution dans 5 ml de chlorure de méthylène sec, sont ajoutés. Le milieu réactionnel est ensuite abandonné sous agitation à la température et pendant le temps indiqués. Le mélange est ensuite hydrolysé en le versant dans 50 ml d'une solution saturée glacée de chlorure d'ammonium à 10 %. La phase organique est séparée par décantation et la phase aqueuse lavée avec 30 ml de pentane. Les phases organiques sont rassemblées et lavées jusqu'à neutralité avec une solution de bicarbonate de sodium, puis séchées sur sulfate de sodium. Après évaporation des solvants, les produits de la réaction sont séparés par chromatographie sur colonne de silice (70-230 Mesh ; 30 g de silice pour 1 g de produit) en éluant avec un mélange chlorure de méthylène/pentane (1/1 dans le cas de l'acétylation et 3/1 dans celui de la benzoylation).

| Chlorure d'acide | T° (C) | | Durée (h) | Rendement |
|---|---|---|---|---|
| | Addition | Réaction | | |
| MeCOCl | - 80 | - 40 | 3 | 80 |
| PhCOCl | 20 | reflux | 18 | 52 |

### 1.3.2 - Réaction de bromation

Appareillage :

Ballon de 50 ml muni d'une agitation magnétique et d'un réfrigérant ascendant.

Mode opératoire :

0,16 g (0,001 mol) de brome sont additionnés à 0,17 g (0,001 mol) de fluorobenzène (disilicié) en solution dans 5 ml de dibromométhane à température ambiante. Le mélange réactionnel est ensuite chauffé à 80°C pendant 3 heures ; puis il est hydrolysé avec 10 ml d'eau et extrait avec 3x5 ml d'éther di isopropylique.

On obtient 51 % de orthobromofluorobenzène et 4 % de méta triméthylsilyl fluorobenzène par analyse par chromatographie en phase gazeuse et par résonance magnétique nucléaire.

### 1.3.3 - Ioduration

Appareillage :

Tube SCHOTT.

Mode opératoire :

0,001 mol de monochlorure d'iode sont ajoutés à 0,001 mol d'aromatique disilicié en solution dans 2 ml de $CH_2Br_2$ à température ambiante. Le mélange est ensuite chauffé dans le cas du fluorobenzène disilicié à 80°C pendant 10 heures puis hydrolysé et extrait avec $CH_2Br_2$.

On obtient après analyse par chromatographie en phase gazeuse et résonance magnétique nucléaire 32 %

d'ortho iodofluorobenzène et 15 % de métatriméthyl silylfluorobenzène.

1.3.4 - Nitrosation

Mode opératoire :

A 0,2405 g (0,001 mol) de fluorobenzène disilicié sont ajoutés 0,15 g (0,001 mol) de $AlCl_3$ en solution dans 10 ml $CH_2Cl_2$ et NOCl gazeux à température ambiante. Le mélange est ensuite chauffé à 40° C pendant 3 heures ; puis hydrolysé et extrait au $CH_2Cl_2$. .

Après analyse par chromatographie en phase gazeuse, on obtient 4 % de nitroso-2 triméthylsilyl-3 fluorobenzène.

1.4 - DESILYLATION DU FLUORO-3, ACYL-4, TRIMETHYLSILYLBENZENE

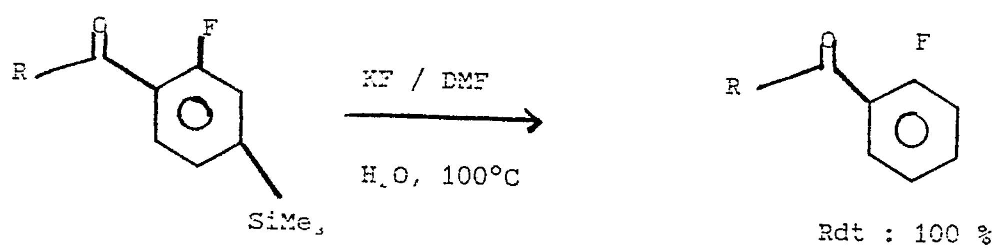


Appareillage :

Ballon de 50 ml muni d'un réfrigérant ascendant.

Mode opératoire :

A 0,5 g (2,4 mmol) de fluoro-3, acétyl-4, triméthylsilylbenzène en solution dans 15 ml de DMF sont ajoutés 0,21 g (3,5 mmol) de fluorure de potassium et 0,04 g (2,4 mmol) d'eau. Le mélange est chauffé à 100°C pendant 18 heures ; après retour à température ambiante, il est versé dans 20 ml d'eau. La phase aqueuse est extraite avec 50 ml de pentane et les phases organiques sont rassemblées et lavées avec 50 ml d'eau, puis séchées sur $Na_2SO_4$. Après évaporation du solvant, l'acétyl-2 fluorobenzène est obtenu avec un rendement quantitatif.

La désilylation du fluoro-3, benzoyl-4, triméthylsilylbenzène est réalisée selon le même protocole opératoiire.

EXEMPLE 2

On reproduit l'exemple 1 en remplaçant le fluorobenzène par l'éthylbenzène.

2.1. - La double silylation en ortho et en méta donne un rendement en bis(triméthylsilyl)-3,6 éthylcyclohexadiène-1,4 de 75 %.

2.2.1 L'aromatisation est effectuée à l'air comme dans la partie 1.2.2 de l'exemple 1 pendant 36 heures et fournit un rendement en bis(triméthylsilyl)-2,5 éthylbenzène de 64 %.

2.2.2 L'aromatisation effectuée dans le tétrachlorure de carbone en présence de rayonnements UV pendant 40 heures donne un rendement en bis(triméthylsilyl)-2,5 éthylbenzène de 63 %.

2.2.3 Par action de p-chloranile : une solution de 5 g (20 mmol) de dérivé cyclohexadiénique et de 10,3 g de p-chloranile dans 200 ml de toluène est chauffée au reflux pendant 40 heures.

Après évaporation du toluène, le résidu est filtré sur un verre fritté rempli de silice et élué avec 150 ml de pentane. Après élimination du solvant et distillation, nous avons obtenu le produit avec 33 % de rendement.

2.3. - FONCTIONNALISATION DU BIS(TRIMETHYLSILYL)-2,5 ETHYLBENZENE

2.3.1 - Réaction d'acrylation

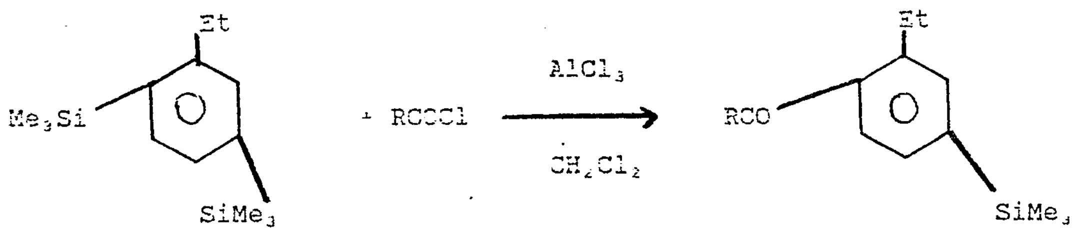


En opérant dans le même appareillage qu'à l'exemple 1.3.1, on met en oeuvre
0,001 mol d'$AlCl_3$, dans 2 ml de $CH_2Br_2$
0,001 mol de chlorure de benzoyle
puis on ajoute 0,001 mol d'éthylbenzène disilicié. Le mélange est chauffé à 50°C pendant 5 heures. Il est ensuite hydrolysé (10 ml d'eau) et extrait avec $CH_2Br_2$(15 ml).

Après analyse par chromatographie en phase gazeuse, on obtient 36 % de benzoyl-6, triméthylsilyl-3, éthylbenzène et 4 % de benzoyl-2, triméthylsilyl-3, éthylbenzène.

2.3.2 - Ioduration

On utilise le même appareillage que dans l'exemple 1.3.3 en mettant en oeuvre les mêmes quantités de produit. Le mélange est chauffé à 50°C pendant 5 heures puis traité comme dans l'exemple 1.3.3.

On obtient 45 % d'iodo-6, triméthylsilyl-3, éthylbenzène.

2.3.3 - Nitration

Appareillage :

Ballon de 50 ml muni d'une agitation magnétique et d'une ampoule à solide, l'ensemble étant sout atmosphère inerte.

Mode opératoire :

0,1704 (0,001 mol) de $NO_2BF_4$ sont ajoutés à 0,2550 g (0,001 mol) d'éthylbenzène disilicié en solution dans 5 ml de $CH_2Cl_2$ à 0°C. Le mélange est agité à température ambiante durant 2 heures ; on hydrolyse (10 ml d'eau) puis on extrait au $CH_2Cl_2$.

Après analyse par chromatographie en phase gazeuse, on détecte la présence de nitro-6, triméthylsilyl-3 éthylbenzène.

2.3.4 - Cyanuration

Appareillage :

Tube SCHOTT

Mode opératoire :

A 0,2511 g (0,001 mol) d'éthylbenzène disilicié en solution dans 2 ml de $CH_2Br_2$ sont ajoutés 0,1512 g (0,001 mol) d'isocyanate de chlorosulfonyle. Le mélange est chauffé pendant 5 heures à 80°C. Après retour à température ambiante 0,1 ml de DMF sont additionnés, l'agitation est maintenue à température ambiante pendant 2 heures. Le milieu réactionnel est ensuite hydrolysé et extrait à l'éther di isopropylique.

Après analyse par chromatographie en phase gazeuse, on obtient 14 % de cyano-6, triméthylsilyl-3, éthylbenzène.

**Revendications**

1. Procédé de substitution en position ortho de fluoro- ou d'alkylbenzènes caractérisé en ce qu'on met en contact
dans une première étape un alkyl ou du fluorobenzène avec un halogénotrialkylsilane en présence d'un métal alcalin ou de magnésium ;
dans une deuxième étape, on aromatise le dérivé obtenu à la première étape
dans une troisième étape on condense sur le dérivé disilylé un agent électrophile
dans une quatrième étape on réalise une désilylation.

2. Procédé selon la revendication 1 caractérisé en ce que, l'agent électrophile est choisi parmi les acides, les halogénures d'acides, les anhydrides d'acides, le chlorosulfonate de triméthylsilyle, les tétrafluoroborates de nitronium ou de nitrosonium, le chlorure de tertiobutyle.

3. Procédé selon la revendication 2 caractérisé en ce que les acides et leurs dérivés sont choisis parmi les acides carboxyliques et les acides sulfoniques.

4. Procédé selon la revendication 3 caractérisé en ce que la condensation est effectuée en présence

d'un acide de Lewis.

5. Procédé selon la revendication 4 caractérisé en ce que l'acide de Lewis est choisi parmi le chlorure d'aluminium, le trifluorure de bore, le tétrachlorure de titane, le tétrachlorure d'étain.

6. Procédé selon la revendication 1 caractérisé en ce que la désilylation des fluoro- ou des alkylbenzènes obtenus à la troisième étape est effectuée par un fluorure alcalin ou alcalinoterreux, un hydroxyde alcalin, d'ammonium, un alcoolate, un ammonium quaternaire, l'acide trifluoroacétique, l'acide trifluorométhane sulfonique.

7. Procédé selon la revendication 1 caractérisé en ce que la 1ère étape est effectuée dans un solvant aprotique polaire tel que le tétrahydrofuranne.

8. Procédé selon la revendication 1 caractérisé en ce que dans la 1ère étape le métal alcalin choisi est le lithium.

9. Procédé selon les revendications 1 et 2 caractérisé en ce que la 4ème étape est effectuée dans un solvant choisi parmi le diméthylacétamide, le diméthylformamide, la N-méthylpyrrolidone-2, le diméthylsulfoxyde,les solvants aliphatiques ou aromatiques éventuellement halogénés.